# EUROPÄISCHE PATENTSCHRIFT

(11) **EP 2 949 638 B1**
(45) Veröffentlichungstag und Bekanntmachung des Hinweises auf die Patenterteilung: **08.11.2017**
(21) Anmeldenummer: 15168685.4
(22) Anmeldetag: 21.05.2015
(51) Int. Cl.: C07C 37/11, C07C 39/15

(54) **VERFAHREN ZUR HERSTELLUNG VON 2,2 -BIPHENOLEN UNTER VERWENDUNG VON SELENDIOXID**
METHOD FOR THE PREPARATION OF 2,2 -BIPHENOLS USING SELENIUM DIOXIDE
DISPOSITIF DE PRODUCTION DE 2,2 BIPHÉNOLS À L'AIDE D'ANHYDRIDE SÉLÉNIEUX

(30) Priorität: 26.05.2014 DE 102014209967
(43) Veröffentlichungstag der Anmeldung: 02.12.2015
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Waldvogel, Siegfried R., 55435 Gau-Algesheim (DE); Quell, Thomas, 55118 Mainz (DE); Mirion, Michael, 55122 Mainz (DE)

(56) Entgegenhaltungen:
- WO-A1-2010/023258
- BEI-SIH LIAO ET AL: "Efficient oxidative coupling of 2,6-disubstituted phenol catalyzed by a dicopper(ii) complex", DALTON TRANSACTIONS, Bd. 41, Nr. 4, 1. Januar 2012 (2012-01-01) , Seiten 1158-1164, XP55143000, ISSN: 1477-9226, DOI: 10.1039/c1dt11065a
- BERND ELSLER ET AL: "Metal- and Reagent-Free Highly Selective Anodic Cross-Coupling Reaction of Phenols", ANGEWANDTE CHEMIE INTERNATIONAL EDITION, 18. März 2014 (2014-03-18), Seiten n/a-n/a, XP55203477, ISSN: 1433-7851, DOI: 10.1002/anie.201400627

## Beschreibung

Die Erfindung betrifft ein Verfahren zur Herstellung von 2,2'-Biphenolen unter Verwendung von Selendioxid.

Die direkte Kupplung von Phenolen zu den entsprechenden Biphenolderivaten, die von großem industriellen Interesse sind, stellt nach wie vor eine Herausforderung dar, da diese Reaktionen oftmals weder regio- noch chemoselektiv sind.

Der Begriff Phenole wird in dieser Anmeldung als Gattungsbegriff verwendet und umfasst somit auch substituierte Phenole.

Die Synthese dieser Biphenole ist u.a. mittels elektrochemischer Verfahren möglich. Hierbei wurden Kohlenstoffelektroden wie Graphit, Glaskohlenstoff, BDD oder Edelmetalle wie Platin verwendet (vgl. F. Stecker, A. Fischer, I. M. Malkowsky, S. R. Waldvogel, A. Kirste, WO 2010139687 A1 und A. Fischer, I. M. Malkowsky, F. Stecker, S. R. Waldvogel, A. Kirste WO 2010023258 A1). Nachteiteilig bei diesen elektrochemischen Verfahren sind die zum Teil teuren Apparaturen, die extra angefertigt werden müssen. Weiterhin ist eine Hochskalierung auf den Tonnenmaßstab, wie er in der Industrie üblicherweise benötigt wird, zum Teil sehr aufwändig und in einigen Fällen sogar unmöglich.

Die direkte Kreuzkupplung ungeschützter Phenolderivate unter klassischen organischen Bedingungen ist bisher nur bei wenigen Beispielen gelungen. Hierfür wurden meist überstöchiometrische Mengen an anorganischen Oxidationsmitteln wie AlCl₃, FeCl₃, MnO₂ oder das organische DDQ verwendet (vgl. G. Sartori, R. Maggi, F. Bigi, M. Grandi, J. Org. Chem. 1993, 58, 7271).

Alternativ werden solche Kupplungsreaktionen in einer mehrstufigen Sequenz durchgeführt. Hierbei werden Abgangsfunktionalitäten und oft giftige, komplizierte Übergangsmetallkatalysatoren, welche beispielsweise auf Palladium basieren, verwendet.

Ein großer Nachteil der oben genannten Methoden zur Phenol-Kupplung ist die Notwendigkeit trockener Lösungsmittel und des Luftausschlusses. Beides bedeutet einen großen Aufwand, gerade, wenn das Verfahren großtechnisch eingesetzt werden soll.

Des Weiteren treten bei den im Stand der Technik beschriebenen Reaktionen oft toxische Nebenprodukte auf, die vom gewünschten Produkt aufwendig abgetrennt und teuer entsorgt werden müssen. Durch knapper werdende Rohstoffe (z.B. Bor und Brom) und die steigende Relevanz des Umweltschutzes steigt der Preis für solche Transformationen. Vor allem bei der Nutzung von mehrstufigen Sequenzen ist ein Wechsel von verschiedenen Lösungsmitteln notwendig, was einen hohen Aufwand darstellt und einen zusätzlichen Kostenfaktor bedeutet.

Der Erfindung lag die Aufgabe zugrunde, ein Verfahren bereitzustellen, welches die in Zusammenhang mit dem Stand der Technik beschriebenen Nachteile nicht aufweist. Insbesondere soll ein Verfahren bereitgestellt werden, mit welchem 2,2'-Biphenolen selektiv hergestellt werden können, also bei der Herstellung möglichst wenig Nebenprodukte anfallen. Das Verfahren sollte auch großtechnisch einsetzbar sein.

Gelöst wird die Aufgabe durch ein Verfahren nach Anspruch 1.

Verfahren zur Herstellung von 2,2'-Biphenolen umfassend die Verfahrensschritte:
a) Zugabe eines ersten Phenols zum Reaktionsgemisch,
b) Zugabe eines zweiten Phenols zum Reaktionsgemisch,
c) Zugabe von Selendioxid zum Reaktionsgemisch,
d) Zugabe einer Säure mit einem pKs-Wert im Bereich von 0,0 bis 5,0 zum Reaktionsgemisch,
e) Erwärmen des Reaktionsgemisches, so dass das erste Phenol und das zweite Phenol zu einem 2,2'-Biphenol umgesetzt werden.

Die Schritte a) bis d) können hierbei in beliebiger Reihenfolge durchgeführt werden.

Das Verfahren ist nicht auf die oben dargestellten Komponenten beschränkt. Weitere Bestandteile wie beispielsweise Lösungsmittel können ebenfalls in dem Reaktionsgemisch enthalten sein.

Verfügt die Säure über mehr als einen pKs-Wert, so ist der pKs₁-Wert heranzuziehen. Dieser muss im erfindungsgemäßen Fall im Bereich von 0,0 bis 5,0 liegen. Bei dem pKs-Wert handelt es sich um den Wert des Moleküls, wie es unter neutralen (also weder sauren noch basischen) Bedingungen vorliegt, und nicht um den Wert der korrespondierenden Säure (pKca). In einem neutralen Umfeld ist also beispielsweise Pyridin als nicht protoniert anzusehen. Die Definition des pKs-Wertes bzw. pKb-Wertes ist dem Fachmann hinlänglich bekannt und kann der entsprechenden Fachliteratur entnommen werden.

Ein Problem beim Einsatz von Selendioxid ist, dass der entsprechende 2,2'-Selenobiarylether und das entsprechende Pummerer-Keton in großen Mengen als Nebenprodukt anfallen können. Bei ungünstiger Reaktionsführung kann es sogar vorkommen, dass der 2,2'-Selenobiarylether das Hauptprodukt der Reaktion darstellt. Gemäß der Aufgabenstellung der Erfindung gilt es jedoch die Reaktion gerade so zu führen, dass jene Nebenprodukte möglichst reduziert werden.

Durch Zugabe von Selendioxid als Oxidationsmittel können abhängig von den Reaktionsbedingungen 2,2'-Biphenole oder 2,2'-Selenobiarylether als Hauptprodukte der Reaktion anfallen (vgl. Schema 1).

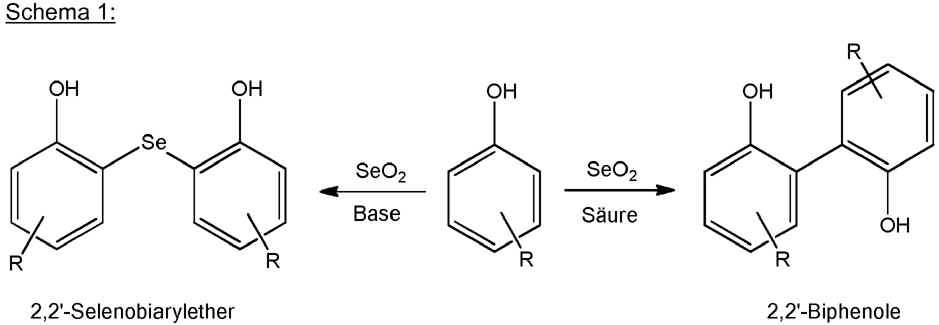

Es wurde gefunden, dass sich die Reaktion durch Zugabe einer Säure mit einem pKs-Wert im Bereich von 0,0 bis 5,0 gezielt in die Richtung des 2,2'-Biphenole verschieben lässt.

Weitere Vorteile gegenüber der im Stand der Technik beschriebenen Verfahren sind, dass dabei nicht unter Feuchtigkeits- oder Sauerstoffausschluss gearbeitet werden muss. Dies stellt einen deutlichen Vorteil gegenüber anderen Syntheserouten dar. Diese direkte Methode der C,C-Kupplung ist ein effizientes und selektives Verfahren, welches sich in vorteilhafter Weise von den bestehenden mehrstufigen Syntheserouten abhebt.

Über den pKs-Werte kann die Reaktion in Richtung C,C-Kupplung gelenkt werden. Dadurch, dass überwiegend das gewünschte Hauptprodukt gebildet wird und die Bildung von höhermolekularen Überoxidationsprodukten verringert wird, wird die Aufarbeitung deutlich vereinfacht.

Nicht umgesetzte Edukte sowie eingesetzte Lösungsmittel können destillativ zurückgewonnen und für weitere Reaktionen verwendet werden. Somit erfüllt das erfindungsgemäße Verfahren die Erfordernisse eines ökonomischen, großtechnischen Verfahrens.

Ferner wird im erfindungsgemäßen Verfahren Selendioxid verwendet. Selendioxid ist ein Abfallprodukt aus der Metallaufreinigung und Erzraffination. Somit wird in dem hier beanspruchten Verfahren ein Abfallprodukt aus anderen Prozessen erneut wertschöpfend eingesetzt. Dies ist insbesondere vor dem Hintergrund der Nachhaltigkeit von Prozessen ein wichtiges Thema.

In einer Variante des Verfahrens handelt es sich bei dem ersten Phenol in Verfahrensschritt a) um eine Verbindung gemäß der allgemeinen Formel **I:** 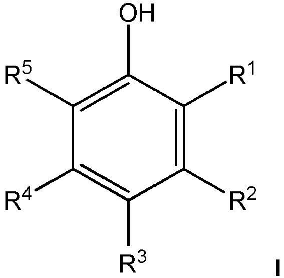 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens R¹ oder R⁵ gleich -H ist.
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind aus:
(C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und O-(C₆-C₂₀)-Aryl können jeweils unsubstituiert oder durch einen oder mehrere gleiche oder verschiedene Reste substituiert sein, die ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck (C₁-C₁₂)-Alkyl geradkettige und verzweigte Alkylgruppen. Vorzugsweise handelt es sich dabei um unsubstituierte geradkettige oder verzweigte (C₁-C₈)-Alkylgruppen und ganz bevorzugt (C₁-C₆)-Alkylgruppen. Beispiele für (C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte (C₁-C₁₂)-Alkylgruppen und substituierte (C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt sind aus: -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,

In einer Variante des Verfahrens sind R¹, R², R³, R⁴, R⁵ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R¹ oder R⁵ gleich -H ist.

In einer Variante des Verfahrens sind R¹, R², R³, R⁴, R⁵ jeweils unabhängig voneinander ausgewählt aus:
-H, -(C₁-C₁₂)-Alkyl,
wobei die genannten Alkyl- und Arylgruppen substituiert sein können,
und mindestens R¹ oder R⁵ gleich -H ist.

In einer Variante des Verfahrens sind R¹, R³, R⁵ jeweils unabhängig voneinander ausgewählt aus:
-H, -(C₁-C₁₂)-Alkyl,
wobei die genannten Alkylgruppen substituiert sein können,
und mindestens R¹ oder R⁵ gleich -H ist.

In einer Variante des Verfahrens stehen R² und R⁴ für -H.

In einer Variante des Verfahrens handelt es sich bei dem zweiten Phenol in Verfahrensschritt b) um eine Verbindung gemäß der allgemeinen Formel **II**: 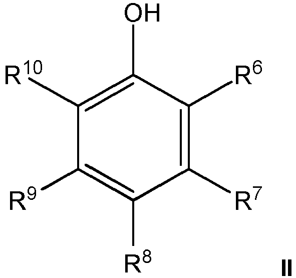 wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen (wie Cl, F, Br, I), -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können, und mindestens R⁶ oder R¹⁰ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R⁶ oder R¹⁰ gleich -H ist.

In einer Variante des Verfahrens sind R⁶, R⁷, R⁸, R⁹, R¹⁰ jeweils unabhängig voneinander ausgewählt aus:
-H, -(C₁-C₁₂)-Alkyl,
wobei die genannten Alkyl- und Arylgruppen substituiert sein können,
und mindestens R⁶ oder R¹⁰ gleich -H ist.

In einer Variante des Verfahrens sind R⁶, R⁸, R¹⁰ jeweils unabhängig voneinander ausgewählt aus:
-H, -(C₁-C₁₂)-Alkyl,
wobei die genannten Alkylgruppen substituiert sein können,
und mindestens R⁶ oder R¹⁰ gleich -H ist.

In einer Variante des Verfahrens stehen R⁷ und R⁹ für -H.

In einer Variante des Verfahrens entspricht das erste Phenol dem zweiten Phenol.

Bei dieser Variante handelt es sich also um eine Homokupplung zweier gleicher Phenole. Durch die ortho-ortho-Kupplung entstehen so die gewünschten 2,2'-Biphenole.

In einer Variante des Verfahrens wird das Selendioxid in Verfahrensschritt c) in einem molaren Verhältnis bezogen auf die Summe des ersten und des zweiten Phenols zugegeben, welches in einem Bereich von 0,25 bis 1,2 liegt.

Hierbei ist der Bereich von 0,25 bis 0,9 bevorzugt, und der Bereich von 0,4 bis 0,7 besonders bevorzugt.

Dass das Selendioxid in einer unterstöchiometrischen Menge eingesetzt werden kann, stellt einen weiteren Vorteil gegenüber der im Stand der Technik beschriebenen Reaktion mit anderen anorganischen Oxidationsmitteln wie beispielsweise AlCl₃, FeCl₃ oder MnO₂ dar.

In einer Variante des Verfahrens ist die Säure im Verfahrensschritt d) ausgewählt aus: Essigsäure (pKs 4,8), Ameisensäure (pKs 3,8), Trifluoressigsäure (pKs 0,23), Propionsäure (pKs 4,9), Phosphorsäure (pKs₁ 2,1).

In einer Variante des Verfahrens wird die Säure im Verfahrensschritt d) als Lösungsmittel eingesetzt.

In einer Variante des Verfahrens wird das Reaktionsgemisch im Verfahrensschritt e) auf eine Temperatur in dem Bereich von 50 °C bis 110 °C erwärmt.

Hierbei ist der Bereich von 60 °C bis 100 °C bevorzugt, und der Bereich von 70 °C bis 90 °C besonders bevorzugt.

Bei den hierbei angegebenen Temperaturen handelt es sich um die im Ölbad gemessenen Temperaturen.

In einer Variante des Verfahrens erfolgt das Erwärmen im Verfahrensschritt e) über einen Zeitraum im Bereich von 5 Minuten bis 24 Stunden.

Hierbei ist der Bereich von 15 Minuten bis 2,5 Stunden bevorzugt, und der Bereich von 15 Minuten bis 2,0 Stunden besonders bevorzugt.

Im Folgenden wird die Erfindung anhand von Ausführungsbeispielen näher erläutert.

### Analytik

### NMR-Spektroskopie

Die NMR-spektroskopischen Untersuchungen wurden an Multikernresonanzspektrometern des Typs AC 300 oder AV II 400 der Firma *Bruker,* Analytische Messtechnik, Karlsruhe, durchgeführt. Als Lösungsmittel wurde CDCl₃ verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschrift

8.2 mmol des jeweiligen Phenols werden im entsprechenden Lösungsmittel gelöst (8.2 m). Das Reaktionsgemisch wird erhitzt und 4.9 mmol Selendioxid werden unter rühren zugegeben. Das Lösungsmittel wird im Vakuum destilliert (Temperatur <70° C). Eine Fritte wird mit 2,5 cm Kieselgel (unten) und 2,5 cm Zeolith (oben) vorbereitet. Der Destillationsrückstand wird im Laufmittel aufgenommen und auf die Filtrationssäule geben. Mit Cyclohexan:Essigester (95:5) wird das Produkt von der Fritte waschen und in Fraktionen gesammelt. Die Fraktionen die Produkt enthalten werden zusammengefasst und destillativ vom Laufmittel befreit.

Die erhaltenen Fraktionen werden aus Cyclohexan:Essigester 95:5 umkristallisiert. Dazu wird der feste Rückstand bei 50 °C gelöst und unlösliche Rückstände über eine Glasfritte abfiltriert. Aus der gesättigten Lösung kristallisiert das Reaktionsprodukt bei Raumtemperatur über Nacht. Die erhaltenen Kristalle werden nochmal mit kaltem Cyclohexan gewaschen.

Die Strukturformel zeigt jeweils das bei der Reaktion angefallene Hauptprodukt.

### 3,3',5,5'-Tetramethylbiphenyl-2,2'-diol

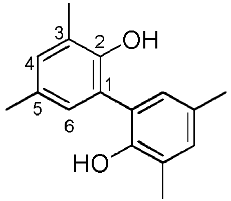

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Säure gelöst und erhitzt. Das Produkt wird als beiger kristalliner Feststoff erhalten.
¹H-NMR (300 MHz, CDCl₃):
δ (ppm) = 7.00 (s,2H, 6-H), 6.87 (s, 2H, 4-H), 5.07 (s,2H, OH), 2.27 (s, 12H, 3-CH₃, 5-CH₃).
¹³C-NMR (75 MHz, CDCl₃):
δ (ppm) = 149.2 (C-2),132.1 (C-4), 130.0 (C-5), 128.5 (C-6), 125.1 (C-3), 122.1 (C-1), 20.4 (5-CH₃), 16.2 (3-CH₃).

### Bis(3,5-dimethyl-2-hydroxyphenyl)selen

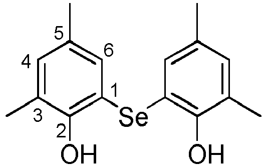

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Pyridin gelöst und erhitzt. Das Produkt wird als farbloser kristalliner Feststoff erhalten.
¹H-NMR (400 MHz, CDCl₃):
δ (ppm) = 7.12 (s,2H, 6-H), 6.91 (s, 2H, 4-H), 5.97 (s,2H, OH), 2.23 (s, 6H, 3-CH₃) 2.23 (s, 6H, 5-CH₃).
¹³C-NMR (100 MHz, CDCl₃):
δ (ppm) = 151.7 (C-2),133.2 (C-3), 133.1 (C-5), 130.4 (C-4), 124.2 (C-6), 114.9 (C-1), 20.3 (5-CH₃), 16.5 (3-CH₃).
⁷⁷Se-NMR (76 MHz, CDCl₃):
δ (ppm) = 163.36 ppm.
Bis(3-*tert*-butyl-5-methyl-2-hydroxyphenyl)selen

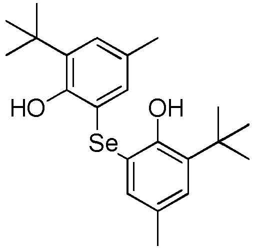

Die Durchführung der Reaktion erfolgt gemäß der allgemeinen Arbeitsvorschrift in einem verschraubbaren Reagenzglas. Dazu wurden 1.32 g (8.0 mmol, 1.0 Äquiv.) 2-*tert*-Butyl-4-methylphenol und 0.54 g (4.9 mmol, 0.6 Äquiv.) Selendioxid in 1 mL Pyridin gelöst und erhitzt.
¹H-NMR (300 MHz, CDCl₃):
δ (ppm) = 7.15 (s, 2H, 6-H), 7.05 (s, 2H, 4-H), 5.07 (s,2H, OH), 2.21 (s, 6H, 5-CH₃), 2.21 (s, 18H, 3-C(CH₃)₃.
¹³C-NMR (75 MHz, CDCl₃):
δ (ppm) = 152.1, 136.4, 133.4, 120.1, 129.5, 117.2, 35.1, 29.6, 20.8.

### 3,3'-Di-tert-butyl,5,5'-dimethylbiphenyl-2,2'-diol

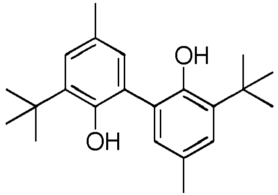

Die Durchführung der Reaktion erfolgt gemäß der allgemeinen Arbeitsvorschrift in einem verschraubbaren Reagenzglas. Dazu wurden 5.00 g (30.5 mmol, 1.0 Äquiv.) 2-*tert*-Butyl-4-methylphenol und 2.03 g (18.3 mmol, 0.6 Äquiv.) Selendioxid in 5 mL Essigsäure gelöst und erhitzt.
¹H-NMR (400 MHz, CDCl₃):
δ (ppm) = 7.17 (d, J=2.2 Hz, 2H), 6.91 (d, J=2.2 Hz, 2H), 5.19 (s, 2H), 2.33 (s, 6H), 1.45 (s, 18H).
¹³C-NMR (75 MHz, CDCl₃):
δ (ppm) = 149.9, 137.0, 129.7, 128.9, 128.6, 122.7, 35.0, 29.8, 27.0.

### Bis(3,5-Di-tert-butyl-2-hydroxyphenyl)selen

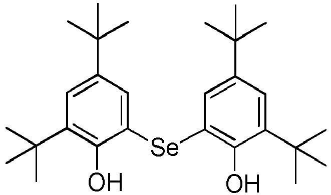

¹H-NMR (400 MHz, CDCl₃):
δ (ppm) = 7.31 (d, J=2.4 Hz, 2H), 7.29 (d, J=2.4), 6.29 (s, 2H), 1.42 (s, 18H), 1.24 (s, 18H).
¹³C-NMR (75 MHz, CDCl₃):
δ (ppm) = 151.7, 143.5, 135.8, 129.8, 125.6, 117.2, 35.4, 34.4, 31.6, 29.7.

### 3,3',5,5'-Tetra-tert-butylbiphenyl-2,2'-diol

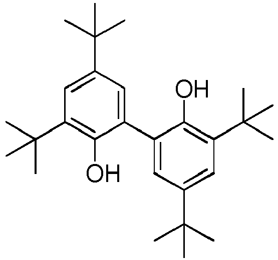

In einem 100 mL Rundkolben wurden 9.700 g 2,4-Di-tert-butylphenol (47 mmol) in 65 mL Essigsäure gelöst, mit 3.130 g Selendioxid (28 mmol) versetzt und im 100 °C heißen Ölbad erhitzt. Nach 30 Minuten wurde die Reaktion beendet, mit 250 mL Essigsäureethylester verdünnt und filtriert. Das Filtrat wurde dreimal mit je 150 mL Wasser gewaschen, die organische Phase abgetrennt, über Magnesiumsulfat getrocknet und vom Lösungsmittel befreit. Das Rohprodukt wurde mittels Säulenchromatographie gereinigt: Die Länge der Säule betrug 8 cm mit einem Durchmesser von 12 cm. Als Eluent wurde Cyclohexan/Essigsäureethylester im Verhältnis 99/1 verwendet. Das so erhaltene Produkt wurde unter Rückfluss in 90 mL Heptan erhitzt, bis es sich vollständige löste. Die Lösung wurde im Ölbad über Nacht bis auf Raumtemperatur abgekühlt. Das so erhaltene Produkt wurde abfiltriert und mit kaltem Heptan gewaschen. Das Filtrat wurde unter vermindertem Druck eingeengt und drei Tage bei 4 °C im Kühlschrank zur Kristallisation gebracht. Es wurden farblose Nadeln erhalten.

Ausbeute: 5.459 g (13.3 mmol), 56%
¹H-NMR (400 MHz, CDCl₃): δ (ppm) =7.39 (d, J=2.4 Hz, 2H), 7.11 (d, J=2.4, 2H), 5.21 (s, 2H), 1.45 (s, 18H), 1.32 (s, 18H).
¹³C-NMR (75 MHz, CDCl₃): δ (ppm) = 149.9, 143.0, 125.4, 124.9, 122.4, 35.4, 34.6, 31.7, 29.8.

### 3,3'-Diisopropyl-5,5',6,6'-tetramethyl-2,2'-biphenol.

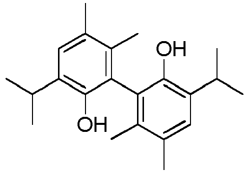

In einem 50 mL Rundkolben wurden 860 mg 4,5-Dimethyl-2-isopropylphenol (5 mmol) in 20 mL Essigsäure gelöst, mit 581 mg Selendioxid (5 mmol) versetzt und im 105 °C heißen Ölbad erhitzt. Nach 40 Minuten wurde die Reaktion beendet, mit 100 mL Essigsäureethylester verdünnt und filtriert. Das Filtrat wurde dreimal mit je 50 mL Wasser gewaschen, die organische Phase abgetrennt und über Magnesiumsulfat getrocknet. Es wurden 872 mg Rohprodukt erhalten, welches mittels Kugelrohrdestillation gereinigt wurde, wobei das Produkt bei 0.001 mbar und 74 °C sublimierte. Es wurden 351 mg farbloser, klarer Kristalle erhalten. Trotz Temperaturerhöhung konnte das Produkt nicht vollständig sublimiert werden, sodass das Gaschromatogramme des Destillationssumpfes geringe Spuren des Produktes zeigte. Zur Probenvorbereitung werden die jeweiligen Proben über eine Kieselgel (ca. 4 cm) gefüllte Pasteurpipette mit filtriert. Der größte Teils des Destillationssumpfes blieb auf dem Kieselgel zurück.

Ausbeute: 351mg (1.0 mmol), 41%
¹H-NMR: (400 MHz, CDCl3) δ [ppm] = 1.25 (dd, 12H), 1.85(s, 6H), 2.26 (s, 6H), 3.26(hept, J=6.9 Hz, 2H), 4.60(s, 2H), 7.06(s, 2H);
¹³C-NMR: (100 MHz, CDCl3) δ [ppm] =16.21, 20.11, 22.72, 22.87, 27.19, 120.34, 128.24, 128.74, 132.12, 133.81, 149.04;
Schmelzbereich: 108.8-109.7 °C

### 3,3'-Di-tert-butyl-5,5',6,6'-tetramethyl-2,2'-biphenol

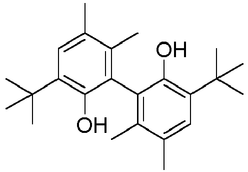

In einem 100 mL Rundkolben wurden 10.719 g 2-*tert*-Butyl-4,5-dimethylphenol (60 mmol) in 35 mL Essigsäure gelöst, mit 4.003 g Selendioxid (36 mmol) versetzt und im 80 °C heißen Ölbad erhitzt. Nach 75 Minuten wurde die Reaktion beendet, mit 250 mL Essigsäureethylester verdünnt und filtriert. Das Filtrat wurde dreimal mit je 150 mL Wasser gewaschen, die organische Phase abgetrennt und über Magnesiumsulfat getrocknet. Das Rohprodukt wurde über eine Schicht aus 3cm Kieselgel und 2 cm Celite filtriert, wobei ein Gemisch aus Cyclohexan/Essigsäureethylester 95/5 als Eluent diente. Das Filtrat wurde vom Lösungsmittel befreit und unter Rückfluss in Heptan erhitzt. Langsames Abkühlen auf Raumtemperatur über Nacht führte zu klaren, leicht gelblichen Nadeln. Das Produkt wurde abfiltriert und mit kaltem Heptan gewaschen. Das Filtrat wurde auf die Hälfte eingeengt und bei 4 °C über zwei Tage zur Kristallisation gebracht.

Ausbeute: 5.004 g (14.1 mmol), 47%
¹H-NMR: (400 MHz, CDCl₃) δ [ppm] = 1.40 (s, 18H), 1.83(s, 6H), 2.26 (s, 6H), 4.81(s, 2H), 7.14(s 2H);
Schmelzbereich: 165.2-165.5 °C.

### 3,3',5,5',6,6'-Hexamethyl-2,2'-biphenol

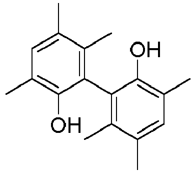

In einem 10 mL Rundkolben wurden 512 mg 2,4,5-Trimethylphenol (4 mmol) in 5 mL Essigsäure gelöst, mit 229 mg Selendioxid (2 mmol) versetzt und im 80 °C heißen Ölbad erhitzt. Nach 120 Minuten wurde die Reaktion beendet, mit 50 mL Dichlormethan verdünnt und filtriert. Das Filtrat wurde dreimal mit je 50 mL Wasser gewaschen, die organische Phase abgetrennt, über Magnesiumsulfat getrocknet und vom Lösungsmittel befreit. Das Rohprodukt wurde Säulenchromatographisch gereinigt: Die Säulenlänge betrug 10 cm und der Säulendurchmesser 3 cm. Es wurde ein Eluent aus Cyclohexan/ Essigsäureethylester 95/5 verwendet. Das gereinigte Produkt wurde bei 60 °C in 5 mL Heptan gelöst. Kristallisation bei 4 °C ergab nach zwei Tagen farblose, nadelförmige Kristalle. Das Produkt wurde abfiltriert, mit kaltem Heptan gewaschen du unter Vakuum von Lösungsmittelresten befreit.

Ausbeute: 226 mg (0.8 mmol), 47%
¹H-NMR: (400 MHz, CDCl₃) δ [ppm] = 1.40 (s, 18H), 1.83(s, 6H), 2.26 (s, 6H), 4.81(s, 2H), 7.14(s 2H);
¹³C-NMR:(75 MHz, CDCl₃) δ [ppm] = 15.73, 16.07 19.51, 119.96, 121.38, 128.48, 132.67, 133.87, 149.79

Schmelzbereich: 68.8-169.5 °C

Die Ergebnisse der oben beschrieben Reaktion, und Variationen derselben, sind in den folgenden Tabellen dargestellt. Die erfindungsgemäßen Verfahren sind hierbei mit * gekennzeichnet.

Folgende Verbindungsklassen werden in den Tabellen näher spezifiziert:

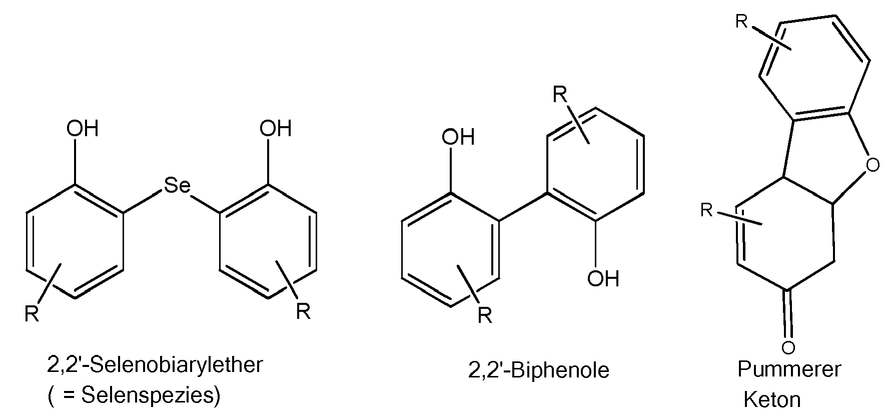

**Tabelle 1a: Oxidative Kupplung von 2,4-Dimethylphenol**

| **basische Bedingungen** | | | | | | |
|---|---|---|---|---|---|---|
| 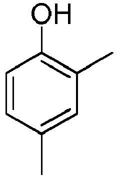 | | | | | | |

| Lösungsmittel | T [°C] | t [h] | pKb-Wert | Pummerer Keton [%] | Biphenol [%] | Selenspezies [%] |
|---|---|---|---|---|---|---|
| Pyridin | 60 | 5 | 8,9 | - | - | 79,1 |
| Pyridin | 85 | 5 | 8,9 | 2,6 | 13,1 | 59,6 |
| Pyridin | 100 | 0,5 | 8,9 | 1,9 | 11,0 | 39,9 |
| Triethylamin (trocken) | 80 | 4 | 3,3 | - | - | 1,8 |
| DMF | 85 | 5 | -1,1 | 4,2 | 19,1 | 18,8 |

Der Tabelle 1a kann man entnehmen, dass (mit Ausnahme von Dimethylformamid (DMF)) das gewünschte Biphenol lediglich als Nebenprodukt anfällt. Bei DMF fallen das Biphenol und die unerwünschte Selenspezies etwa zu gleichen Teilen an.

**Tabelle 1b: Oxidative Kupplung von 2,4-Dimethylphenol**

| **saure Bedingungen** | | | | | | |
|---|---|---|---|---|---|---|
| 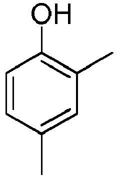 | | | | | | |

| Lösungsmittel | T [°C] | t [h] | pKs-Wert | Pummer er Keton [%] | Biphen ol [%] | Selenspezi es [%] |
|---|---|---|---|---|---|---|
| Essigsäure* | 85 | 5 | 4,8 | 4,5 | 74,8 | 1,98 |
| Essigsäure* | 60 | 1,5 | 4,8 | 1,8 | 39,8 | 8,0 |
| Trifluoressigsäure / Essigsäure* (3:1) | 85 | 5 | 0,23 / 4,8 | 2,5 | 77,8 | 1,4 |
| Ameisensäure* | 60 | 2 | 3,8 | 1,8 | 85,4 | - |
| Methansulfonsäure | 85 | 5 | -2,6 | - | 3,9 | 6,1 |
| p-Toluolsulfonsäure | 85 | 5 | -2,8 | - | 15,0 | - |

Der Tabelle 1b kann man entnehmen, dass unter den erfindungsgemäßen sauren Bedingungen jeweils das Biphenol in sehr guter Ausbeute hergestellt werden konnte. Unter Verwendung von Methansulfonsäure (pKs -2,6) und p-Toluolsulfonsäure (pKs -2,8), deren pKs-Werte jeweils unter dem Wert von 0,0 liegen, konnte das gewünschte Biphenol nur in sehr geringen Ausbeuten erhalten werden.

**Tabelle 2a: Oxidative Kupplung von 2,4-Di-tert-butylphenol**

| **basische Bedingungen** | | | | | |
|---|---|---|---|---|---|
| 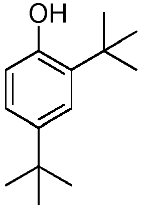 | | | | | |

| Lösungsmittel | T [°C] | t [h] | pKb-Wert | Biphenol [%] | Selenspezies [%] |
|---|---|---|---|---|---|
| Pyridin | 40 | 24 | 8,9 | 20,6 | 46,8 |
| Pyridin | 60 | 7 | 8,9 | 10,1 | 30,4 |

Unter basischen Bedingungen entsteht wieder die Selenspezies als Hauptprodukt der Reaktion.

**Tabelle 2b: Oxidative Kupplung von 2,4-Di-tert-butylphenol**

| **saure Bedingungen** | | | | | |
|---|---|---|---|---|---|
| 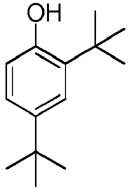 | | | | | |

| Lösungsmittel | T [°C] | t [h] | pKs-Wert | Biphenol [%] | Selenspezies [%] |
|---|---|---|---|---|---|
| Essigsäure* | 50 | 18 | 4,8 | 29,5 | 25,2 |
| Essigsäure* | 85 | 1 | 4,8 | 25,9 | 23,1 |
| Essigsäure* | 105 | 0,2 | 4,8 | 75,1 | 2,6 |
| Ameisensäure* | 70 | 1 | 3,8 | 46,9 | 7,6 |

Unter den erfindungsgemäßen sauren Bedingungen ist das angestrebte Biphenol das Hauptprodukt der Reaktion.

**Tabelle 3a: Oxidative Kupplung von 2-tert-Butyl-4-methylphenol**

| **basische Bedingungen** | | | | | |
|---|---|---|---|---|---|
| 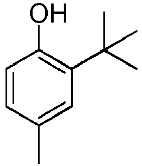 | | | | | |

| Lösungsmittel | T [°C] | t [h] | pKb-Wert | Biphenol [%] | Selenspezies [%] |
|---|---|---|---|---|---|
| Pyridin | 40 | 24 | 8,9 | 7,2 | 61,5 |
| Pyridin | 60 | 7 | 8,9 | 1,6 | 32,2 |
| Pyridin | 85 | 1,5 | 8,9 | 6,1 | 32,5 |
| Pyridin | 100 | 0,5 | 8,9 | 4,5 | 28,9 |

Auch aus der Tabelle 3a ist wiederum ersichtlich, dass basische Bedingungen zur Selenspezies führen, und das angestrebte Biphenol nur zu sehr geringen Anteilen erhalten wird.

**Tabelle 3b: Oxidative Kupplung von 2-tert-Butyl-4-methylphenol**

| **saure Bedingungen** | | | | | |
|---|---|---|---|---|---|
| 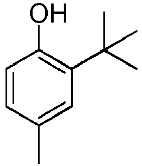 | | | | | |

| Lösungsmittel | T [°C] | t [h] | pKs-Wert | Biphenol [%] | Selenspezies [%] |
|---|---|---|---|---|---|
| Essigsäure* | 50 | 18 | 4,8 | 34,2 | 19,8 |
| Essigsäure* | 85 | 1,5 | 4,8 | 63,7 | 4,0 |
| Essigsäure* | 100 | 0,4 | 4,8 | 53,2 | 2,1 |

Unter erfindungsgemäßen Bedingungen hingegen kann das angestrebte Biphenol in sehr guten Ausbeuten hergestellt werden.

Die in den Tabellen 1a bis 3b zusammengefassten Ergebnisse zeigen eindeutig, dass das erfindungsgemäße Verfahren die zuvor definierte Aufgabenstellung erfüllt. Das erfindungsgemäße Verfahren stellt eine Syntheseroute dar, mit welcher 2,2'-Biphenole selektiv, in guter Ausbeute hergestellt werden können. Des Weiteren lässt sich das erfindungsgemäße Verfahren auch großtechnisch verwirklichen.

Im Folgenden werden weitere Vergleichsversuche beschrieben.

Es wurde 2,4-Dimethylphenol mit SeO₂ umgesetzt.

**Tabelle 4:**

| Übersicht der Umsetzung von 2,4-Dimethylphenol mit Selendioxid bei verschiedenen Temperaturen und Lösungsmitteln nach 18 Stunden. Es wurden jeweils 1,3 Äquivalente Selendioxid (bezogen auf 2,4-Dimethylphenol) eingesetzt. | | | | | | |
|---|---|---|---|---|---|---|
| Lösungsmittel | T [°C] | t [h] | 2,4-Dimethyl phenol | Pummerer -Keton | Biphen ol | Selens pezies |
| THF | 90 | 18 | 98 | 1 | 1 | - |
| Glyme | 96 | 18 | - | 17 | 74 | - |
| Glyme | 75 | 18 | 13 | 9 | 52 | 10 |
| Diglyme | 96 | 18 | 95 | 1 | 4 | - |

Verwendet man Glyme (Ethylenglycoldimethylether) als Lösungsmittel, so erhält man auch hier das Biphenol als Hauptprodukt. Es bedarf allerdings hoher Temperaturen und langer Reaktionszeiten, was die Reaktion für einen großtechnischen Einsatz unattraktiv macht. Ein weiterer Nachteil ist, dass das Selendioxid überstöchiometrisch, also mit mehr als 1,0 Äquivalenten, eingesetzt werden muss. Des Weiteren liegt sie Summe der Nebenprodukte (Pummerer-Keton + Selenspezies) jeweils über 15%. Durch den hohen Anteil an Nebenkomponenten wird die entsprechende Aufarbeitung zur Gewinnung der Reinsubstanz deutlich schwieriger und damit verbunden auch teurer, was für einen großtechnischen Prozess unvorteilhaft ist.

Mit Tetrahydrofuran (THF) (pKb 11,5) als Lösungsmittel bildet sich so gut wie kein Biphenol.

Unter Einsatz des erfindungsgemäßen Verfahrens können in viel kürzerer Zeit deutlich bessere Reaktionsergebnisse erzielt werden.

## Patentansprüche

1. Verfahren zur Herstellung von 2,2'-Biphenolen umfassend die Verfahrensschritte:
a) Zugabe eines ersten Phenols zum Reaktionsgemisch,
b) Zugabe eines zweiten Phenols zum Reaktionsgemisch,
c) Zugabe von Selendioxid zum Reaktionsgemisch,
d) Zugabe einer Säure mit einem pKs-Wert im Bereich von 0,0 bis 5,0 zum Reaktionsgemisch,
e) Erwärmen des Reaktionsgemisches, so dass das erste Phenol und das zweite Phenol zu einem 2,2'-Biphenol umgesetzt werden.

2. Verfahren nach Anspruch 1,
wobei es sich bei dem ersten Phenol in Verfahrensschritt a) um eine Verbindung gemäß der allgemeinen Formel **I** handelt: 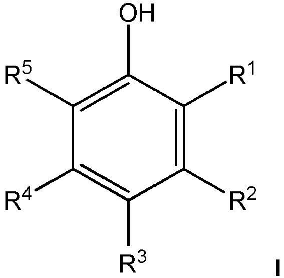 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens R¹ oder R⁵ gleich -H ist.

3. Verfahren nach Anspruch 2,
wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R¹ oder R⁵ gleich -H ist.

4. Verfahren nach einem der Ansprüche 2 oder 3,
wobei R¹, R³, R⁵ jeweils unabhängig voneinander ausgewählt sind aus:
-H, -(C₁-C₁₂)-Alkyl,
wobei die genannten Alkylgruppen substituiert sein können,
und mindestens R¹ oder R⁵ gleich -H ist.

5. Verfahren nach einem der Ansprüche 1 bis 4,
wobei es sich bei dem zweiten Phenol in Verfahrensschritt b) um eine Verbindung gemäß der allgemeinen Formel **II** handelt: 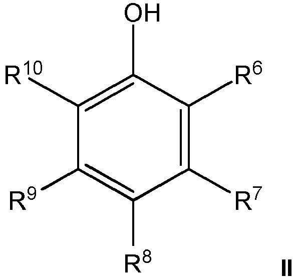 wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl,
zwei benachbarte Reste können des Weiteren zu einem kondensierten System miteinander verbunden sein,
wobei die genannten Alkyl- und Arylgruppen substituiert sein können,
und mindestens R⁶ oder R¹⁰ gleich -H ist.

6. Verfahren nach Anspruch 5,
wobei R⁶, R⁷, R⁸, R⁹, R¹⁰ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl,
wobei die genannten Alkyl- und Arylgruppen substituiert sein können,
und mindestens R⁶ oder R¹⁰ gleich -H ist.

7. Verfahren nach einem der Ansprüche 5 oder 6,
wobei R⁶, R⁸, R¹⁰ jeweils unabhängig voneinander ausgewählt sind aus:
-H, -(C₁-C₁₂)-Alkyl,
wobei die genannten Alkylgruppen substituiert sein können,
und mindestens R⁶ oder R¹⁰ gleich -H ist.

8. Verfahren nach einem der Ansprüche 1 bis 7,
wobei das erste Phenol dem zweiten Phenol entspricht.

9. Verfahren nach einem der Ansprüche 1 bis 8,
wobei das Selendioxid in Verfahrensschritt c) in einem molaren Verhältnis bezogen auf die Summe des ersten und des zweiten Phenols zugegeben wird, welches in einem Bereich von 0,25 bis 1,2 liegt.

10. Verfahren nach einem der Ansprüche 1 bis 9,
wobei die Säure im Verfahrensschritt d) ausgewählt ist aus:
Essigsäure, Ameisensäure, Trifluoressigsäure, Propionsäure, Phosphorsäure.

11. Verfahren nach einem der Ansprüche 1 bis 10,
wobei die Säure im Verfahrensschritt d) als Lösungsmittel eingesetzt wird.

12. Verfahren nach einem der Ansprüche 1 bis 11,
wobei das Reaktionsgemisch im Verfahrensschritt e) auf eine Temperatur in dem Bereich von 50 °C bis 110 °C erwärmt wird.

13. Verfahren nach einem der Ansprüche 1 bis 12,
wobei das Erwärmen im Verfahrensschritt e) über einen Zeitraum im Bereich von 5 Minuten bis 24 Stunden erfolgt.

## Claims

1. Process for preparing 2,2'-biphenols, comprising the process steps of:
a) adding a first phenol to the reaction mixture,
b) adding a second phenol to the reaction mixture,
c) adding selenium dioxide to the reaction mixture,
d) adding an acid having a pKa in the range from 0.0 to 5.0 to the reaction mixture,
e) heating the reaction mixture such that the first phenol and the second phenol are converted to a 2,2'-biphenol.

2. Process according to Claim 1,
wherein the first phenol in process step a) is a compound of the general formula I: 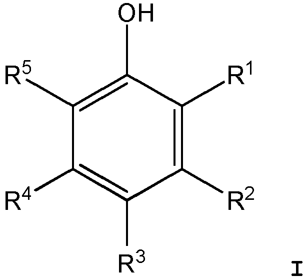 where R¹, R², R³, R⁴, R⁵ are each independently selected from:
-H, -(C₁-C₁₂)-alkyl, -O-(C₁-C₁₂)-alkyl, -(C₆-C₂₀)-aryl, -O- (C₆-C₂₀)-aryl, -halogen, -OC=O-(C₁-C₁₂)-alkyl,
two adjacent radicals may additionally be joined to one another to form a condensed system,
where the alkyl and aryl groups mentioned may be substituted,
and at least R¹ or R⁵ is -H.

3. Process according to Claim 2,
where R¹, R², R³, R⁴, R⁵ are each independently selected from:
-H, -(C₁-C₁₂)-alkyl, -O-(C₁-C₁₂)-alkyl, -(C₆-C₂₀)-aryl, -O-(C₆-C₂₀)-aryl,
where the alkyl and aryl groups mentioned may be substituted,
and at least R¹ or R⁵ is -H.

4. Process according to either of Claims 2 and 3, where R¹, R³, R⁵ are each independently selected from:
-H, -(C₁-C₁₂)-alkyl,
where the alkyl groups mentioned may be substituted, and at least R¹ or R⁵ is -H.

5. Process according to any of Claims 1 to 4,
wherein the second phenol in process step b) is a compound of the general formula II: 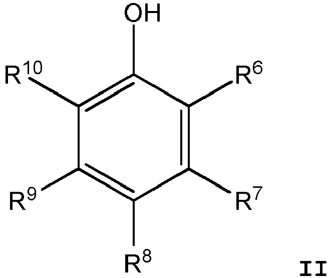 where R⁶, R⁷, R⁸, R⁹, R¹⁰ are each independently selected from:
-H, -(C₁-C₁₂)-alkyl, -O-(C₁-C₁₂)-alkyl, - (C₆-C₂₀)-aryl, -O- (C₆-C₂₀)-aryl, -halogen, -OC=O-(C₁-C₁₂)-alkyl,
two adjacent radicals may additionally be joined to one another to form a condensed system,
where the alkyl and aryl groups mentioned may be substituted,
and at least R⁶ or R¹⁰ is -H.

6. Process according to Claim 5,
where R⁶, R⁷, R⁸, R⁹, R¹⁰ are each independently selected from:
-H, -(C₁-C₁₂)-alkyl, -O-(C₁-C₁₂)-alkyl, -(C₆-C₂₀)-aryl, -O-(C₆-C₂₀)-aryl,
where the alkyl and aryl groups mentioned may be substituted,
and at least R⁶ or R¹⁰ is -H.

7. Process according to either of Claims 5 and 6, where R⁶, R⁸, R¹⁰ are each independently selected from:
-H, -(C₁-C₁₂)-alkyl,
where the alkyl groups mentioned may be substituted, and at least R⁶ or R¹⁰ is -H.

8. Process according to any of Claims 1 to 7,
wherein the first phenol corresponds to the second phenol.

9. Process according to any of Claims 1 to 8,
wherein the selenium dioxide is added in process step c) in a molar ratio based on the sum total of the first and second phenols within a range from 0.25 to 1.2.

10. Process according to any of Claims 1 to 9,
wherein the acid in process step d) is selected from:
acetic acid, formic acid, trifluoroacetic acid, propionic acid, phosphoric acid.

11. Process according to any of Claims 1 to 10, wherein the acid is used as solvent in process step d).

12. Process according to any of Claims 1 to 11, wherein the reaction mixture is heated in process step e) to a temperature in the range from 50°C to 110°C.

13. Process according to any of Claims 1 to 12, wherein the heating in process step e) is effected over a period in the range from 5 minutes to 24 hours.

## Revendications

1. Procédé pour la préparation de 2,2'-diphénols, comprenant les étapes de procédé :
a) addition d'un premier phénol au mélange réactionnel,
b) addition d'un deuxième phénol au mélange réactionnel,
c) addition de dioxyde de sélénium au mélange réactionnel,
d) addition d'un acide présentant une valeur de pKa dans la plage de 0,0 à 5,0 au mélange réactionnel,
e) chauffage du mélange réactionnel de manière telle que le premier phénol et le deuxième phénol sont transformés en un 2,2'-diphénol.

2. Procédé selon la revendication 1,
le premier phénol dans l'étape de procédé a) étant un composé selon la formule générale I : 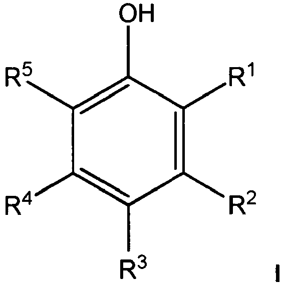 dans laquelle R¹, R², R³, R⁴, R⁵ sont choisis, à chaque fois indépendamment les uns des autres, parme :
-H, -(C₁-C₁₂)-alkyle, -O-(C₁-C₁₂)-alkyle, -(C₆-C₂₀)-aryle, -O- (C₆-C₂₀)-aryle, -halogène, -OC=O-(C₁-C₁₂)-alkyle,
deux radicaux adjacents peuvent en outre être liés l'un à l'autre en un système condensé,
les groupes alkyle et aryle mentionnés pouvant être substitués,
et au moins R¹ ou R⁵ représentant -H.

3. Procédé selon la revendication 2,
R¹, R², R³, R⁴, R⁵ étant choisis, à chaque fois indépendamment les uns des autres, parme :
-H, -(C₁-C₁₂)-alkyle, -O-(C₁-C₁₂)-alkyle, -(C₆-C₂₀)-aryle, -O- (C₆-C₂₀)-aryle,
les groupes alkyle et aryle mentionnés pouvant être substitués,
et au moins R¹ ou R⁵ représentant -H.

4. Procédé selon l'une quelconque des revendications 2 ou 3,
R¹, R³, R⁵ étant choisis, à chaque fois indépendamment les uns des autres, parmi :
-H, -(C₁-C₁₂)-alkyle,
les groupes alkyle mentionnés pouvant être substitués, et au moins R¹ ou R⁵ représentant -H.

5. Procédé selon l'une quelconque des revendications 1 à 4,
le deuxième phénol dans l'étape de procédé b) étant un composé selon la formule générale II : 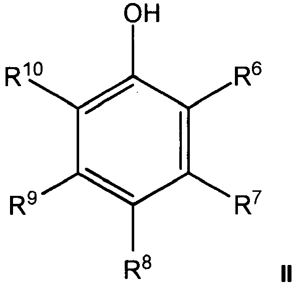 dans laquelle R⁶, R⁷, R⁸, R⁹, R¹⁰ sont choisis, à chaque fois indépendamment les uns des autres, parme :
-H, -(C₁-C₁₂)-alkyle, -O-(C₁-C₁₂)-alkyle, -(C₆-C₂₀)-aryle, -O- (C₆-C₂₀)-aryle, -halogène, -OC=O-(C₁-C₁₂)-alkyle,
deux radicaux adjacents peuvent en outre être liés l'un à l'autre en un système condensé,
les groupes alkyle et aryle mentionnés pouvant être substitués,
et au moins R⁶ ou R¹⁰ représentant -H.

6. Procédé selon la revendication 5,
R⁶, R⁷, R⁸, R⁹, R¹⁰ étant choisis, à chaque fois indépendamment les uns des autres, parme :
-H, -(C₁-C₁₂)-alkyle, -O-(C₁-C₁₂)-alkyle, -(C₆-C₂₀)-aryle, -O- (C₆-C₂₀)-aryle,
les groupes alkyle et aryle mentionnés pouvant être substitués,
et au moins R⁶ ou R¹⁰ représentant -H.

7. Procédé selon l'une quelconque des revendications 5 à 6,
R⁶, R⁸, R¹⁰ étant choisis, à chaque fois indépendamment les uns des autres, parmi :
-H, -(C₁-C₁₂)-alkyle,
les groupes alkyle mentionnés pouvant être substitués, et au moins R⁶ ou R¹⁰ représentant -H.

8. Procédé selon l'une quelconque des revendications 1 à 7,
le premier phénol correspondant au deuxième phénol.

9. Procédé selon l'une quelconque des revendications 1 à 8,
le dioxyde de sélénium dans l'étape de procédé c) étant ajouté dans un rapport molaire, par rapport à la somme du premier et du deuxième phénol, qui est situé dans une plage de 0,25 à 1,2.

10. Procédé selon l'une quelconque des revendications 1 à 9,
l'acide dans l'étape de procédé d) étant choisi parmi :
l'acide acétique, l'acide formique, l'acide trifluoroacétique, l'acide propionique, l'acide phosphorique.

11. Procédé selon l'une quelconque des revendications 1 à 10,
l'acide dans l'étape de procédé d) étant utilisé comme solvant.

12. Procédé selon l'une quelconque des revendications 1 à 11,
le mélange réactionnel étant chauffé dans l'étape e) à une température dans la plage de 50 à 110°C.

13. Procédé selon l'une quelconque des revendications 1 à 12,
le chauffage dans l'étape de procédé e) ayant lieu sur une période de temps dans la plage de 5 minutes à 24 heures.
